(19) Europäisches Patentamt
European Patent Office
Office européen des brevets

(11) EP 4 717 696 A1

(12) **EUROPEAN PATENT APPLICATION**
published in accordance with Art. 153(4) EPC

(43) Date of publication:
**01.04.2026 Bulletin 2026/14**

(21) Application number: **24811406.8**

(22) Date of filing: **22.05.2024**

(51) International Patent Classification (IPC):
***C07D 487/04*** *(2006.01)* ***A61K 31/5517*** *(2006.01)*
***A61P 25/20*** *(2006.01)*

(52) Cooperative Patent Classification (CPC):
**A61K 31/5517; A61P 25/20; C07D 487/04**

(86) International application number:
**PCT/KR2024/006939**

(87) International publication number:
**WO 2024/242468 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.05.2023 KR 20230066525**
**28.11.2023 KR 20230168330**

(71) Applicant: **Hana Pharm. Co. Ltd.**
**Hwaseong-si, Gyeonggi-do 18608 (KR)**

(72) Inventors:
- **OH, Eun Hye**
  **Suwon-si, Gyeonggi-do 16454 (KR)**
- **KOO, Chang Hui**
  **Ansan-si, Gyeonggi-do 15482 (KR)**
- **SEO, Jung Min**
  **Incheon 21652 (KR)**
- **KIM, Ye Rin**
  **Gumi-si, Gyeongsangbuk-do 39459 (KR)**
- **KIM, Young Woong**
  **Seongnam-si, Gyeonggi-do 13348 (KR)**
- **MIN, Chang Ho**
  **Hwaseong-si, Gyeonggi-do 18602 (KR)**
- **MA, Seong Hee**
  **Guri-si, Gyeonggi-do 11947 (KR)**
- **YOO, Jae Ho**
  **Hwaseong-si, Gyeonggi-do 18497 (KR)**
- **HWANG, Yong Youn**
  **Suwon-si, Gyeonggi-do 16295 (KR)**

(74) Representative: **Schön, Christoph**
**Dr. Schön, Neymeyr & Partner mbB**
**Bavariaring 26**
**80336 München (DE)**

# (54) METHOD FOR PREPARING REMIMAZOLAM BESYLATE WITH HIGH EFFICIENCY

(57) The present invention relates to a method for preparing, from a readily commercially available compound of 3-((3S)-7-bromo-2-((2-hydroxypropyl)amino)-5-(pyridin-2-yl)-3H-benzo[e][1,4]diazepin-3-yl)propionic acid methyl ester, 3-[(4S)-8-bromo-1-methyl-6-(2-pyridinyl)-4H-imidazo[1,2-a][1,4]benzodiazepin-4-yl] propionic acid methyl ester benzenesulfonate (remimazolam besylate) with high efficiency.

**FIGURE 1**

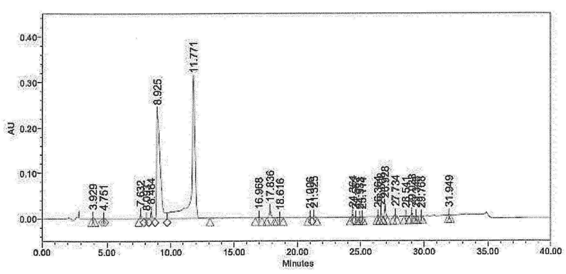


Peak Results

| | Name | RT | Area | % Area | Height | Amount | Units |
|---|---|---|---|---|---|---|---|
| 1 | | 3.929 | 14183 | 0.12 | 2924 | | |
| 2 | | 4.751 | 9851 | 0.08 | 1827 | | |
| 3 | | 7.632 | 78110 | 0.66 | 10750 | | |
| 4 | | 8.093 | 12212 | 0.10 | 1028 | | |
| 5 | | 8.464 | 125238 | 1.06 | 14941 | | |
| 6 | | 8.925 | 4497761 | 37.98 | 233602 | | |
| 7 | | 11.771 | 6340389 | 53.54 | 303333 | | |
| 8 | | 16.968 | 26954 | 0.23 | 2482 | | |
| 9 | | 17.836 | 183647 | 1.55 | 17596 | | |
| 10 | | 18.616 | 6231 | 0.05 | 433 | | |
| 11 | | 21.006 | 38555 | 0.33 | 3589 | | |
| 12 | | 21.325 | 57970 | 0.49 | 5090 | | |
| 13 | | 24.304 | 46019 | 0.39 | 5741 | | |
| 14 | | 24.582 | 14730 | 0.12 | 2011 | | |
| 15 | | 24.933 | 6610 | 0.06 | 1014 | | |
| 16 | | 25.114 | 7821 | 0.07 | 1189 | | |
| 17 | | 26.304 | 19797 | 0.17 | 3535 | | |
| 18 | | 26.608 | 64913 | 0.55 | 10444 | | |
| 19 | | 26.928 | 153806 | 1.30 | 24604 | | |
| 20 | | 27.734 | 46377 | 0.39 | 5625 | | |
| 21 | | 28.541 | 16788 | 0.14 | 1735 | | |
| 22 | | 29.035 | 48194 | 0.41 | 6497 | | |
| 23 | | 29.348 | 8418 | 0.07 | 1896 | | |
| 24 | | 29.768 | 8979 | 0.07 | 1456 | | |
| 25 | | 31.949 | 7810 | 0.07 | 1507 | | |

EP 4 717 696 A1

## Description

### TECHNICAL FIELD

The present invention relates to a method for preparing remimazolam besylate with high efficiency. More specifically, the present invention relates to a method for preparing remimazolam besylate with high efficiency using commercially readily available 3-((3S)-7-bromo-2-((2-hydroxypropyl)amino)-5-(pyridin-2-yl)-3H-benzo[e][1,4]diazepin-3-yl)propionic acid methyl ester (the compound of the following Formula P-3) to synthesize remimazolam besylate (the compound of the following Formula P). More specifically, the present invention provides a method for synthesizing the compound of structure P by oxidizing the compound of Formula P-3 into a mixture of compounds of Formula P-2 and P-1 via a novel oxidation reaction, and then treating this mixture with benzenesulfonic acid.

[Formula P]

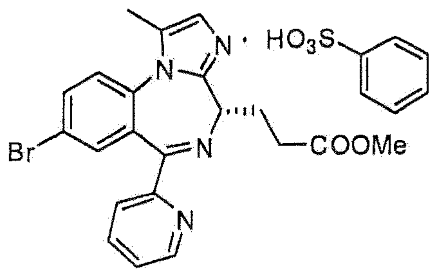

[Formula P-1]

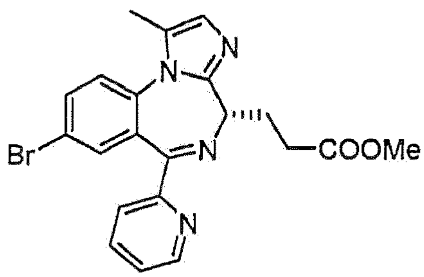

[Formula P-2]

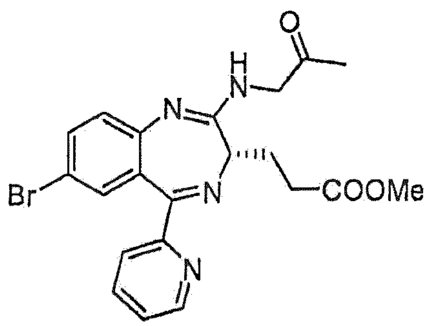

[Formula P-3]

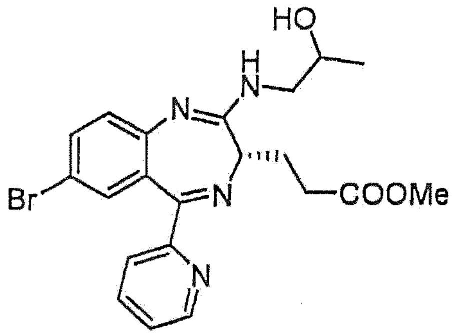

## BACKGROUND ART

Remimazolam besylate is an imidazobenzodiazepine-class psychotropic drug developed by GlaxoSmithKline in the UK and PAION in Germany. It is used as a sedative during surgical anesthesia. Compared to existing propofol, it carries a lower risk of cardiovascular and respiratory depression and is safer for patients with renal and hepatic impairment, drawing attention as a novel anesthetic drug. Remimazolam besylate can be synthesized from the readily available compound P-3 (Formula shown below) via two or three steps, as shown in the scheme of Reaction 1 below.

[Reaction 1]

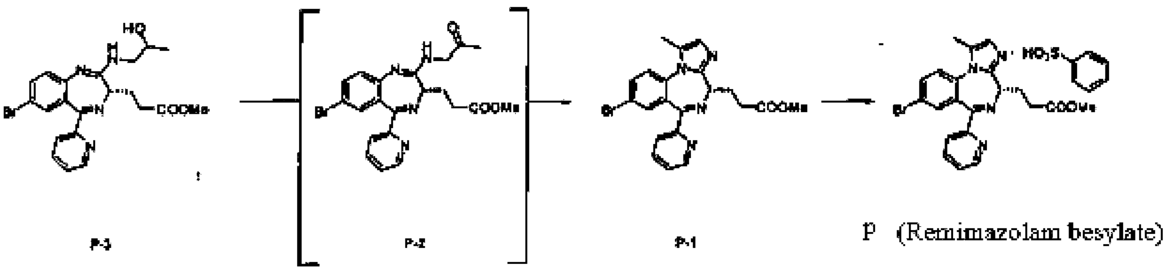

P-3 P-2 P-1 P (Remimazolam besylate)

The oxidation reaction for selectively oxidizing the secondary alcohol of the compound of Formula P-3 to synthesize the compound of Formula P-2 or the compound of Formula P-1, or mixtures thereof, is known as follows.

First, the oxidation reaction using the hypervalent iodine compound (1,1,1-triacetoxy-1,1-dihydro-1,2-benzoiodoxol-3(1H)-one (Dess-Martin periodinane)) for the hypervalent iodine compound (DM) presented in Patent Document 1 can provide compound of Formula P-1, or a mixture thereof, with a yield of 76% via Formula P-2 with a 76% yield and good purity (93.91%). However, Dess-Martin periodinane is an expensive reagent, potentially explosive, and requires 1.5 equivalents of the reactant, making it unsuitable for industrial-scale production due to cost and safety concerns. Furthermore, Swern oxidation using oxalyl chloride in dimethyl sulfoxide (DMSO) and the Albright-Goldman oxidation reagent (acetic anhydride/DMSO) presented in the literature are sensitive to moisture and require ultra-low temperatures below -60°C, necessitating specialized production equipment. Furthermore, the gas byproduct dimethyl sulfide (DMS) poses odor and explosion risks, and the use of DMSO, which is difficult to handle post-reaction, limits its applicability in production processes.

[Reaction 2]

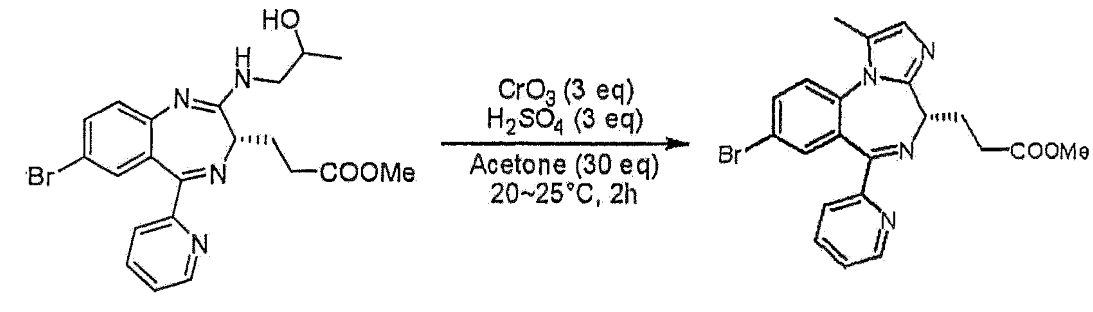


P-3 P-1

The oxidation reaction using chromium trioxide and sulfuric acid described in Patent Document 2 for the above Reaction 2 can synthesize the compound with Formula P-1 with high yield (90%) and purity (99.94%) to synthesize the compound with Formula P-1. However, it uses sulfuric acid, a strong acid, and chromium trioxide, a heavy metal harmful to humans and the environment, making it difficult to apply to pharmaceutical industry processes.

As alternatives to these problems, Patent Document 1, Patent Document 3, and Patent Document 4 propose a method for synthesizing the compound of Formula P-2. This method selectively oxidizes the secondary alcohol of the compound of Formula P-3 using a combination of hypochlorite and an N-oxyl oxidation catalyst. This combination is economical, safe, and does not require special equipment, unlike oxidizing agents that are harmful to humans and the environment or are expensive.

Patent Document 1 provide synthesizing the compound with structure P-2 by using sodium hypochlorite (NaOCl), tetramethylpiperidine-1-oxyl (hereinafter TEMPO), sodium bromide (NaBr), and sodium hydrogen carbonate ($NaHCO_3$) to selectively oxidize the secondary alcohol of the compound with structure P-3. The oxidation conversion rate for this reaction is not documented and thus unknown. However, when subsequently synthesizing the compound of structure P from the compound of structure P-2 in a single step using benzenesulfonic acid, the yield was reported as 50%.

[Reaction 3]

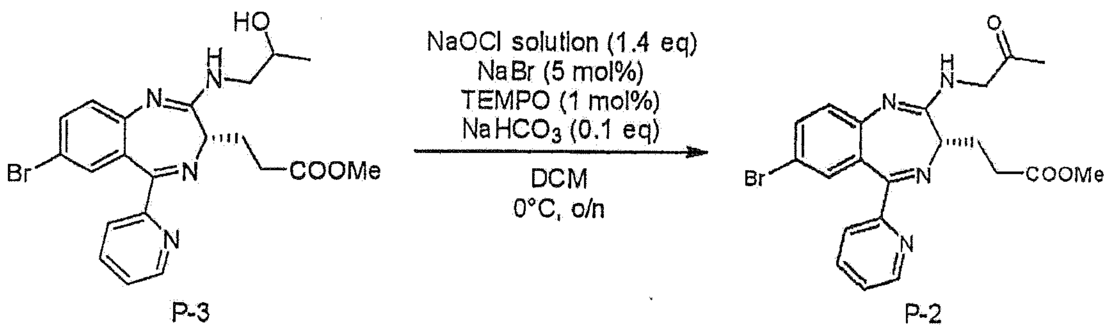


P-3 P-2

Patent Document 3 synthesized the compound of Formula P-2 from the compound of structure P-3 using a combination of sodium hypochlorite (NaOCl) and a catalytic amount of TEMPO, as shown in the following Reaction Equation 4. The subsequent synthesis yield to the besylate was reported as 58.43% (purity: 98.75%).

[Reaction 4]

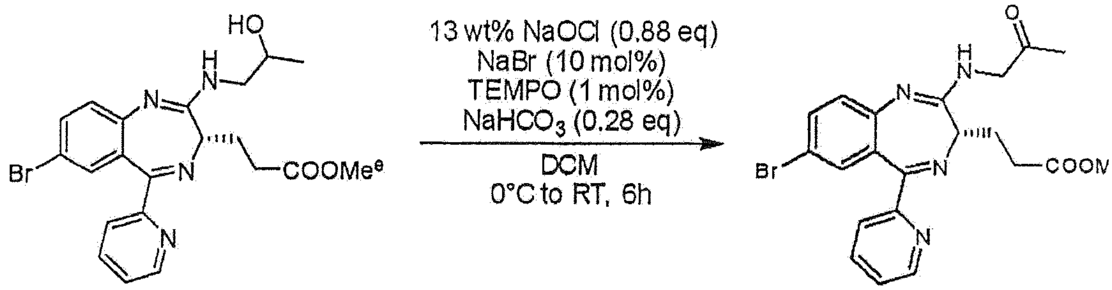


P-3 P-2

Patent Document 4 describes the synthesis of compound P-2 from compound P-3 using sodium hypochlorite (NaOCl) together with 2-azadamantane-N-oxyl (hereinafter AZADO), as shown in the following Reaction 5. The yield is unknown, and the oxidation conversion rate by HPLC is recorded as 99.9%. When directly experimenting under these conditions and analyzing the results according to Test Method 1 described in the following example, the oxidation conversion rate after 1 hour of stirring was low at 32.8%. Stirring was continued for up to 5 hours thereafter, but no further reaction occurred.

[Reaction 5]

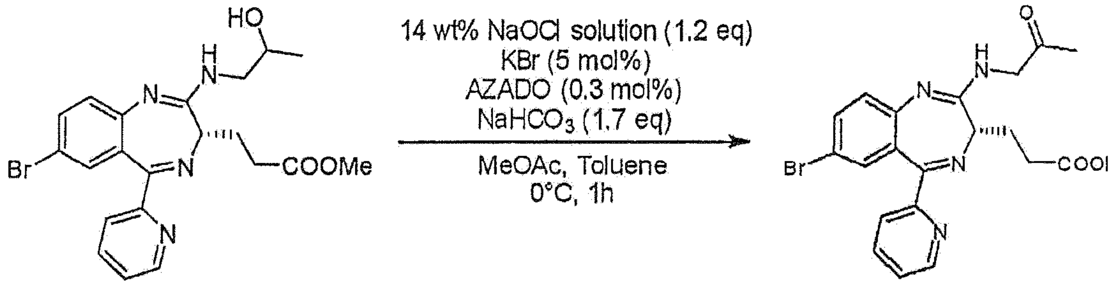


P-3 P-2

The reactions in Patent Document 1, Patent Document 3, and Patent Document 4 have the advantage of performing the oxidation reaction using sodium hypochlorite (NaOCl), an economical and non-toxic oxidizing agent, without requiring special equipment. However, they require maintaining a low temperature for a long time, and the requirement to perform the reaction under basic conditions (pH 8.6-9.5) by adding bases such as sodium bicarbonate ($NaHCO_3$) and salts like sodium or potassium bromide to ensure smooth reaction progress. These basic conditions increase the instability of the compound's ester portion, leading to increased side reactions and impurities. Furthermore, since sodium hypochlorite (NaOCl) in aqueous solution, which is more prone to decomposition compared to its pentahydrate form, is used, this necessitates quantitative analysis via titration prior to use. Due to its aqueous nature, the volume efficiency is typically low, with a maximum sodium hypochlorite (NaOCl) content of 12-13 wt%, resulting in low volumetric efficiency and inevitably increasing the moisture content of the reaction mixture, which heightens the potential for impurity formation.

Consequently, when synthesizing the final substance, remimazolam, from the compound of Formula P-2 obtained by this method, the yield was low at 60% or less, necessitating research into a more efficient process.

One oxidation reaction used to obtain compound P-2 (an intermediate for synthesizing remimazolam besylate, compound P) from compound P-3 involves hypochlorite and N-oxyl oxidizing catalysts (tetramethylpiperidine-1-oxyl (TEMPO) or 2-azadamantane-1-oxyl (AZADO) or their analogues). -N-oxyl (hereinafter AZADO) or its analogues) can safely and economically yield the desired compound with favorable results.

However, these methods require maintaining low temperatures, and as described in Patent Document 5, the reaction proceeds smoothly under basic conditions, necessitating the addition of a base for pH adjustment. However, under these basic conditions, the instability of both the starting material and the product increases due to the nature of remimazolam containing an ester group, potentially leading to the generation of numerous impurities. Consequently, the synthetic yield to the final compound may be low. Indeed, the synthesis yield to the final compound described in Patent

Document 1 and Patent Document 3 was low at 60% or less. The synthesis yield in Patent Document 4 was not reported. When the results were confirmed according to Test Method 1 after directly experimenting based on the patent method, the oxidation conversion rate was low at 32.8%.

Sodium hypochlorite (NaOCl) aqueous solution used in the above documents has a maximum concentration of 13 wt%, resulting in poor volumetric efficiency and low convenience during storage and use. Furthermore, sodium hypochlorite (NaOCl) in aqueous solution is unstable, causing its concentration to continuously decrease, necessitating titration before use. This low storage stability and low volumetric efficiency present disadvantages from an industrial manufacturing perspective, where long-term storage and continuous large-scale use are required. Additionally, using an aqueous solution inevitably introduces a large amount of water into the reaction mixture, changing the pH to 13 and increasing the likelihood of impurity generation. The description of the characteristics of the sodium hypochlorite (NaOCl) aqueous solution above was referenced from Non-Patent Document 1.

## Prior Art

### Patent Documents

[Patent Document 1] KR 10-1801735 B
[Patent Document 2] KR 10-2021-0157146 A
[Patent Document 3] CN 112321594 B
[Patent Document 4] KR 10-2015-0120453 A
[Patent Document 5] KR 10-1162648 B

### Non-Patent Literature

[Non-Patent Literature 1] Organic Process Research & Development 2017 21 (12), 1925-1937
[Non-Patent Document 2] Molecules 2020, 25, 5918

## SUMMARY OF INVENTION

### TECHNICAL PROBLEM

The objective of the present invention is to improve the limitations of existing oxidation reactions in the manufacture of remimazolam besylate from the compound of Formula P-3, thereby obtaining the final target compound (remimazolam besylate) in an economical and safe manner with high efficiency and high yield.

### SOLUTION TO PROBLEM

To resolve the above issues, the inventors recognized the limitations of existing literature synthesizing compound of Formula P-2 from compound of Formula P-3 and sought to improve this by researching oxidation reactions using economical, less harmful to humans, and stable oxidizing agents. Furthermore, by combining and adding catalysts to assist the oxidation reaction, the inventors developed a significantly improved oxidation reaction compared to existing literature. This reaction completes at room temperature in a short time and reduces impurity formation.

Although it is already known from various literature, including Non-Patent Document 1, that sodium hypochlorite pentahydrate ($NaOCl\cdot5H_2O$) is superior to aqueous solutions in terms of oxidizing power, stability, ease of use, and volumetric efficiency, this invention is the first to derive improved results by optimizing and verifying its application to the actual manufacture of remimazolam through multiple experiments.

During the optimization process, alkaline earth metal peroxides and OXONE, which are economical, environmentally friendly, less toxic, and safe oxidizing agents, were used as auxiliary oxidants to assist the oxidation reaction, and ammonium salts known as auxiliary catalysts for oxidation reactions in various literature, including Non-Patent Document 2, were combined and added in appropriate equivalents to induce a low-cost, high-efficiency, and safe oxidation reaction. The application of alkaline earth metal peroxides as catalysts in similar oxidation reactions is novel to this invention. The reason for using alkaline earth metal peroxides is that increasing the amount of oxidizing agents ($NaOCl\cdot5H_2O$, AZADO, or OXONE), increasing the oxidizing agent quantity does not proportionally increase oxidation efficiency. Therefore, using a small amount of this lower-cost catalyst to enhance oxidation efficiency is cost-effective.

Furthermore, alkaline earth metal peroxides belong to the category of mild oxidizing agents. They improve upon the drawbacks of conventional hypochlorite oxidizing agents, such as impurity generation due to rapid oxidation reactions and the necessity of maintaining low temperatures. Simultaneously, they enable reactions with reduced reaction times and improved yields. Calcium peroxide (CaO2), used in the exemplary implementation of this reaction, is an environmentally friendly component also used in toothpaste materials, making its application in pharmaceutical industry processes unproblematic.

Experiments were conducted to discover the combination and optimal usage amounts of oxidizing agents for improving reaction efficiency in the oxidation reaction of the present invention, thereby completing the invention.

Through the solution described above, a reaction was ultimately induced that concludes within 30 minutes at room temperature without requiring low-temperature maintenance, with reduced impurity generation and improved purity and yield. Furthermore, the compound of Formula P-1, a key intermediate in the synthesis of remimazolam besylate, is co-generated during the reaction. This subsequently undergoes a cyclization reaction, inducing a favorable reaction pathway for the synthesis of remimazolam besylate.

## EFFECT OF INVENTION

According to the present invention, the limitations of the conventional reactions described above have been improved by using sodium hypochlorite pentahydrate ($NaOCl \cdot 5H_2O$), an oxidizing agent superior to aqueous solutions in terms of oxidizing power, cost-effectiveness, stability, volumetric efficiency, and ease of use, in the oxidation reaction of the compound of Formula P-3.

Unlike unstable sodium hypochlorite (NaOCl) aqueous solutions, sodium hypochlorite pentahydrate ($NaOCl \cdot 5H_2O$) exhibits minimal concentration variability, eliminating the inconvenience of requiring titration prior to use. It also offers superior volumetric efficiency, with a maximum concentration of 44 wt%, compared to the maximum 12-13 wt% achievable with aqueous solutions. These advantages, including storage stability and volumetric efficiency, are critically important from an industrial manufacturing perspective requiring long-term storage and continuous bulk usage.

Furthermore, while using sodium hypochlorite (NaOCl) aqueous solutions inevitably adds large volumes of water to the reaction mixture and creates a basic environment with a pH of 13, replacing it with sodium hypochlorite pentahydrate ($NaOCl \cdot 5H_2O$), the moisture content of the reaction solution is significantly reduced, and the pH also decreases to 11. As the conditions become relatively milder, the potential for impurity formation decreases, eliminating the need to maintain low temperatures during the reaction. This has led to the invention of reaction conditions that achieve a higher oxidation conversion rate and a higher yield of the target compound.

Furthermore, by identifying the addition and appropriate dosage of alkaline earth metal peroxides-mild oxidizing agents that are economical, environmentally friendly, and less toxic to humans-as auxiliary oxidants, a low-cost, high-efficiency mild oxidation reaction was achieved.

Experiments conducted to determine the optimal dosage of calcium peroxide ($CaO_2$), one of the alkaline earth metal peroxides used in the examples of the present invention, to achieve maximum efficiency showed that the oxidation conversion rate improved significantly at a specific equivalent, rather than increasing proportionally with the amount added. Indeed, comparative experiments under identical conditions with and without calcium peroxide ($CaO_2$) addition, along with dosage optimization, confirmed that the target compound yield increased by over 9% at a specific point. This demonstrates that calcium peroxide ($CaO_2$) acts as an appropriate catalyst when used at the equivalent amount proposed by the inventor for the reaction of the present invention.

The sodium hypochlorite pentahydrate ($NaOCl \cdot 5H_2O$), calcium peroxide ($CaO_2$), OXONE, ammonium salts, and N-oxyl oxidizing catalysts can be used in an appropriate combination to achieve an oxidation conversion rate of 98.9% within 30 minutes at room temperature without the need for low-temperature maintenance or pH adjustment, which were limitations of the prior art. Since basic conditions are unnecessary, the instability of the ester group constituting the compound is resolved, impurities are reduced, and the target compound can be produced with high purity and high yield without purification.

One significant effect of the above combination is that the compound of Formula P-1, a crucial intermediate in the synthesis of remimazolam besylate, is co-generated during the reaction. This facilitates subsequent cyclization reactions, making it advantageous for synthesizing remimazolam besylate. Patent Document 1 and Patent Document 4, which are comparable to the present invention, provide only compounds of Formula P-2 in the N-oxyl oxidant conditions. N-oxyl oxidant conditions only provide compounds of Formula P-2, whereas the embodiments of the present invention provide compounds of Formula P-1 at a high ratio of up to approximately 2:98 relative to compounds of Formula P-2.

The invention was completed by demonstrating that synthesizing the product obtained under the conditions of the present invention into remimazolam besylate resulted in a yield increase of 1.6 to 1.9 times or more for both steps compared to the prior art.

**Brief Description of the Drawings**

Figure 1 shows the reaction mixture after stirring for 30 minutes in Example 3-3, analyzed by HPLC. The peak areas of the compound with Formula P-2, indicated at 8.925 minutes, and the compound with Formula P-1, indicated at 11.771 minutes, account for 91.5% of the total peak area. Their respective ratios are 41:59, indicating greater production of the compound with Formula P-1. The compound with Formula P-3, appearing at 464 minutes, was produced in a small amount at 1.06%, while other impurities accounted for 7.4%.

Figure 2 shows the HPLC analysis of the remimazolam besylate obtained in Example 4. The purity of the remimazolam besylate appearing at 12.553 minutes is 98.49%.

Figure 3 is a graph comparing the results of Comparative Example 1 and Example 1 by temperature.

Figure 4 is a graph showing the target compound yield of Comparative Example 1 and Example 1 conducted at 15°C as a function of reaction time.

Figure 5 is a graph showing the target compound yield of Comparative Example 1 and Example 1 conducted at room temperature as a function of reaction time.

**Best Mode for Carrying Out the Invention**

The present invention will now be described in detail.

The present invention relates to a method for manufacturing remimazolam besylate (Structure P), comprising step (a) oxidizing 3-((3S)-7-bromo-2-((2-hydroxypropyl)amino)-5-(pyridin-2-yl)-3H-benzo[e] [1,4]diazepin-3-yl)propionic acid methyl ester with a hydrated hypochlorite salt, an N-oxyl oxidizing catalyst, an ammonium salt, and an inorganic oxidizing agent in the presence of an organic solvent; and (b) treating the reaction product obtained in step (a) with benzenesulfonic acid in an organic solvent or organic solvent mixture. In this case, the reaction product obtained in step (a) is a mixture of structures (P-1) and (P-2).

[Formula P-3]

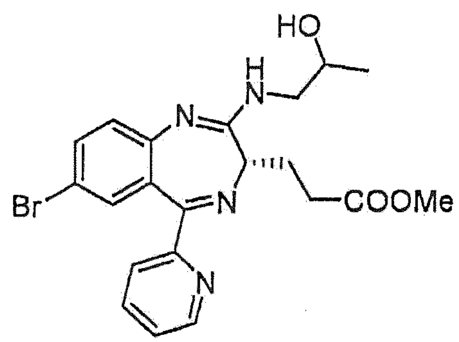

[Formula P-2]

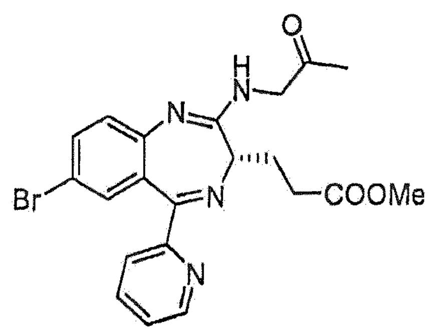

[Formula P-1]

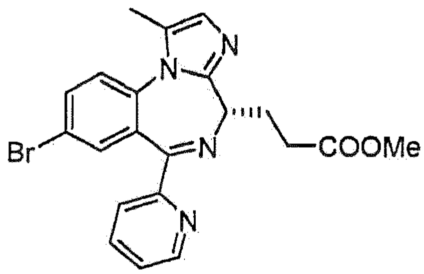

[Formula P]

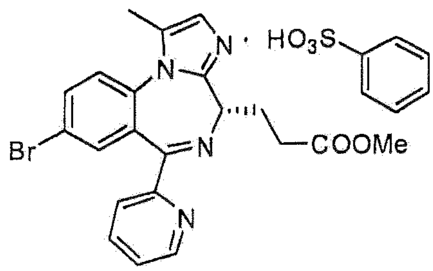

The compound of Formula P-3 is readily commercially available and can be synthesized according to the method described in Patent Document 1.

The reaction in step (a) is the oxidation of the compound of Formula P-3 in the presence of an organic solvent, using a hydrated hypochlorite salt and an N-oxyl oxidizing catalyst. Step (a) may additionally employ ammonium salts, an inorganic oxidizing agent, or a peroxide of an alkaline earth metal. The reaction conditions are such that the oxidizing agent oxidizes the secondary alcohol to a ketone, producing Formula P-2, without reacting with other functional groups of the compound of Formula P-3, while simultaneously allowing some of the compound of Formula P-2 to undergo cyclization under these conditions, converting it to the compound of Formula P-1.

Consequently, the product of step (a) may be a mixture of compounds of Formula P-2 and compounds of Formula P-1.

Compounds of Formula P-1 and compounds of Formula P-2 can be separated by methods commonly used in organic synthesis technology, but the product (mixture) of step (a) can proceed to step (b) without purification after work-up. Since the cyclization to the compound of Formula P-1 and the synthesis of remimazolam besylate of Formula P are performed in a single step under acidic conditions in step (b), the generation of the compound of Formula P-1 is advantageous for the synthesis of the final compound.

For the purpose of the present invention, it is desirable to terminate the reaction when the compound of Formula P-3 is consumed by 90% or more, preferably 95% or more, and more preferably 98% or more, while simultaneously keeping the impurity generation rate at 10% or less, preferably 6% or less.

The reaction is stirred for 15 minutes or longer and completed within 180 minutes; more preferably within 40 minutes; most preferably, stirring for 20 minutes or longer and completing within 35 minutes yields the highest purity product.

The reaction can be performed at room temperature (0°C-30°C) without special temperature settings, and most preferably at 20°C-25°C, which is most appropriate considering the oxidation conversion rate and the potential for impurity generation.

The oxidizing agent in step (a) is a hydrated form of hypochlorite, which may be selected from the group consisting of a hydrated form of sodium hypochlorite (NaOCl), a hydrated form of potassium hypochlorite (KOCl), and the a hydrated form of calcium hypochlorite ($Ca(OCl)_2$), or a mixture of the aforementioned oxidizing agents when required.

Specifically, among the above oxidizing agents, the use of sodium hypochlorite pentahydrate ($NaOCl{\cdot}5H_2O$) is preferred.

The above oxidizing agent may be used in an amount of 0 to 10 equivalents, preferably 0.1 to 5 equivalents, relative to the compound of Formula P-3.

The reaction behavior differs depending on the form of sodium hypochlorite used in the above reaction. When

sodium hypochlorite pentahydrate ($NaOCl \cdot 5H_2O$) was used, it exhibited a higher oxidation conversion rate in the reaction compared to a sodium hypochlorite (NaOCl) aqueous solution (12~14 wt%), and the degree of impurity generation was also significantly reduced.

N-oxyl oxidation catalysts used in step (a) may be any one selected from the group consisting of 2,2,6,6-tetramethylpiperidine N-oxyl (TEMPO), 4-methoxy-TEMPO, 4-ethoxy-TEMPO, 4-acetyloxy-TEMPO, 4-acetamido-TEMPO, 4-hydroxy-TEMPO, 4-benzoyloxy-TEMPO, 4-amino-TEMPO, N,N-dimethylamino-TEMPO, 4-oxo-TEMPO, and its 4-substituted derivatives, and poly[(6-[1,1,3,3-tetramethylbutyl)amino]-s-triazine-2,4-diyl], ]], polymeric versions of TEMPO such as Chimassorb 944 (CAS No. 71878-19-8), also known as poly[6-[(1,1,3,3-tetramethylbutyl)amino]1,3,5-triazine-2,4-diyl]-[(2,2,6,6-tetramethyl-4-piperidyl)-imino]hexamethylene[(2,2,6,6-tetramethyl-4-piperidyl)imino], 2-azadamantane-N-oxyl (AZADO) , 1-methyl-2-azadamantane-N-oxyl (Me-AZADO), 2-hydroxy-2-azadamantane (AZADOH), Nor-AZADO, ABNO, their salts, and their solvates. The N-oxyl oxidation catalyst of step (a) above is a mixture obtained by combining the aforementioned catalysts as required.

N-oxyl oxidation catalyst is preferably specifically one selected from AZADO, Me-AZADO, AZADOH, Nor-AZADO, TEMPO, and ABNO, and more preferably specifically AZADO, Me-AZADO, and ABNO.

The N-oxyl oxidation catalyst can be used in an equimolar ratio of 0.001 to 1, preferably 0.01 to 0.2, relative to the compound of Formula P-3.

The structure of the compound represented by 2-azaadamantane-N-oxyl (AZADO) used in the present invention is as shown in the following Formula 1.

[Formula 1]

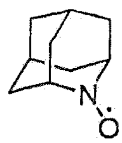

The structure of the compound represented by 1-methyl-2-azadamantane-N-oxyl (Me-AZADO) used in the present invention is as shown in the following Formula 2.

[Formula 2]

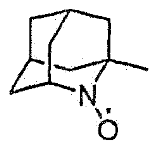

The structure of the compound represented as 2-hydroxy-2-azadamantane (AZADOH) used in the present invention is as shown in the following Formula 3.

[Formula 3]

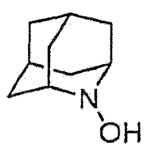

The structure of the compound represented by 2,2,6,6-tetramethylpiperidine N-oxyl (TEMPO) used in the present invention is as shown in the following Formula 4.

[Formula 4]

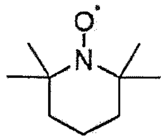

The structure of the compound represented by 9-azabicyclo[3.3.1]nonan-N-oxyl (ABNO) used in the present invention is as shown in the following Formula 5.

[Formula 5]

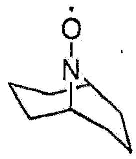

The ammonium salts that assist the oxidation reaction in step (a) may be one selected from the group consisting of tetrabutylammonium bromide (TBAB), tetrabutylammonium fluoride (TBAF), tetrabutylammonium chloride (TBAC), tetrabutylammonium iodide (TBAI); a salt and solvate thereof, and preferably tetrabutylammonium bromide (TBAB).

The ammonium salt may be used in an equimolar ratio of 0.001 to 10, preferably 0.01 to 0.5, most preferably 0.01 to 0.2, relative to the compound of Formula P-3.

The peroxide of an alkaline earth metal assisting the oxidation reaction in step (a) may be one selected from the group consisting of calcium peroxide ($CaO_2$), compounds represented by magnesium peroxide ($MgO_2$), salts thereof, and solvates thereof, and preferably calcium peroxide ($CaO_2$).

The alkaline earth metal peroxide can be used in an amount of 0 to 1 equivalent ratio, preferably 0.01 to 0.5 equivalent ratio, most preferably 0.05 to 0.2 equivalent ratio, relative to the compound of Formula P-3.

The reaction in step (a) differs depending on the addition of calcium peroxide ($CaO_2$). When calcium peroxide ($CaO_2$) was added, the yield of the target compound increased compared to the reaction without addition.

The oxidation conversion rate and target compound yield varied with the amount of calcium peroxide ($CaO_2$) added. Experiments conducted with calcium peroxide ($CaO_2$) added at 0.037 equivalents (3.7 mol%)/0.075 equivalents (7.5 mol%)/0.1 equivalents (10 mol%)/ 0.15 mol% (15 mol%) of calcium peroxide (CaO2). The results showed the optimal outcome at 0.1 mol% (10 mol%), while using more than necessary decreased the oxidation conversion rate. Furthermore, the yield of compound P-1 was highest at this point. This indicates that an optimal amount exists when using calcium peroxide ($CaO_2$) as a catalyst for mild oxidation reactions, and identifying and applying this amount to the reaction is a crucial factor.

The inorganic oxidizing agent assisting the reaction in step (a) may be one selected from the group consisting of the compound represented by commercially available oxone (OXONE, $2KHSO_5.KHSO_4.K_2SO_4$), its salts, and its solvates.

The inorganic oxidizing agent may be used in an amount of 0.1 to 10 equivalents, preferably 0.01 to 3 equivalents, relative to the compound of Formula P-3.

The organic solvent in step (a) may be ethyl acetate, methyl acetate, methylene chloride, dichloroethane, toluene, acetone, diethyl ether, methyl tert-butyl ether, pentane, hexane, acetonitrile, or tetrahydrofuran, or a mixture obtained by combining the aforementioned solvents as required.

Specifically, it is desirable that the organic solvent be one or more selected from ethyl acetate, methylene chloride, acetonitrile, and tetrahydrofuran, and more specifically, excellent results are obtained with acetonitrile.

In the present invention, the term "work-up" in the phrase "the product (mixture) of step (a) can proceed to step 2) without purification after work-up" refers to the general work-up procedure performed with an aqueous solution and an organic solvent for reaction quenching and washing in organic synthesis. In the above reaction, washing and reaction quenching are induced using an aqueous solution of sodium thiosulfate and ethyl acetate, and the separated organic layer is extracted and dried to proceed to step (b).

In the preparation of remimazolam besylate (the compound of Formula P), the concentrated residue obtained after the work-up of step (a) can be manufactured without performing purification steps such as distillation, silica gel chromatography, or recrystallization. This is because the oxidation reaction in step (a) proceeds with significantly high conversion rates and good reproducibility.

After performing the reaction of step (a), the reaction of step (b) is a step for producing remimazolam besylate

(compound of Formula P) by reacting the concentrated residue obtained from step (a) with an organic acid in the presence of an organic solvent.

The reaction of step (b) is a one-pot reaction comprising the step of cyclizing the compound of Formula P-2 to convert it into the compound of Formula P-1, and the step of forming a salt of the compound of Formula P-1, which exists as the product of step (a) or as converted, to synthesize the remimazolam besylate of Formula P.

The organic acid of step (b) may be one selected from the group consisting of benzenesulfonic acid, its salts, its hydrates, and its solvates.

The organic acid may be used in an equimolar ratio of 0.9 to 2, preferably 0.9 to 1.3, relative to the compound of Formula P-3.

The reaction of step (b) may be carried out at 0°C to 100°C, preferably 20°C to 50°C, for 1 hour to 72 hours, preferably 24 hours to 48 hours.

The organic solvent of step (b) may be methanol, ethanol, 1-propanol, 2-propanol, n-butyl alcohol, t-butyl alcohol, ethyl acetate, methyl acetate, methylene chloride, acetonitrile, or methyl ethyl ketone, or a mixture obtained by combining the aforementioned solvents as required.

The organic solvent is preferably specifically one selected from methanol, ethanol, ethyl acetate, or a mixture thereof.

Remimazolam besylate (compound of Formula P) generated during the reaction in step (b) can be isolated by methods commonly used in organic synthesis. It can be formed as crystals from the reaction mixture during the reaction or separated by crystallization from one selected from methanol, ethanol, ethyl acetate, or a mixture thereof.

## Examples

Information on the main reagents used in the examples described herein is as follows.

Sodium hypochlorite (NaOCl) aqueous solution (hereinafter NaOCl aqueous solution)
Manufacturer: Sigma-Aldrich, Concentration: 10-15 wt%
Sodium hypochlorite pentahydrate (hereinafter $NaOCl \cdot 5H_2O$)
Manufacturer: WAKO, Concentration: 39%
Calcium peroxide ($CaO_2$)
Manufacturer: Sigma-Aldrich, Purity: 75%
2-Azadamantane-N-oxyl (hereinafter AZADO)
Manufacturer: Sigma-Aldrich, Purity: 90%
Oxone (hereinafter OXONE)
Manufacturer: TCI, Purity: >Ca.45% (T) as KHSO5
Tetrabutylammonium bromide (hereinafter TBAB)
Manufacturer: JUNSEI, Purity: 98%
Benzenesulfonic acid
Manufacturer: Sigma-Aldrich, Purity: 90%

HPLC analysis of the examples in the main text was performed under the following conditions.
HPLC Conditions
Column: YMC ODS-AQ, 250 x 4.6 mm, 3 μm particle size
Mobile Phase
Mobile Phase A: Water containing 0.01% trifluoroacetic acid
Mobile Phase B: Acetonitrile containing 0.01% trifluoroacetic acid
Gradient:

| Time (min) | % A | % B |
|---|---|---|
| 0.0 | 75 | 25 |
| 20.0 | 60 | 40 |
| 30.0 | 20 | 80 |
| 32.0 | 20 | 80 |
| 32.5 | 75 | 25 |
| 40.0 | 75 | 25 |

Flow rate: 1.0 mL/min
Column temperature: 40°C
Autosampler: Ambient
Detection: UV at 230 nm
Injection volume: 10 μl
Run time: 40 min

Under the described conditions, the retention time of the compound of Formula P-3 is approximately 8.4 minutes, the retention time of the compound of Formula P-2 is approximately 9.2 minutes, and the retention times of the compound of Formula P-1 and remimazolam besylate are both approximately 11.9 minutes.

The oxidation conversion rate and target compound yield used to derive the results of the examples were calculated according to the methods described in Test Method 1 and Test Method 2 below.

Test Method 1: Calculation Method for Oxidation Conversion Rate

[Calculation Formula 1]

$$\text{Oxidation Conversion Rate} = 100 * \frac{A(P-2)+A(P-1)}{A(P-3)+A(P-2)+A(P-1)}$$

The oxidation conversion rate is a percentage value predicted by HPLC for the ratio of starting material to target compound in the reaction mixture, calculated according to [Calculation Formula 1], without considering impurities.

A(P-3) represents the peak area of the compound with Formula P-3;
A(P-2) represents the peak area of the compound with Formula P-2;
A(P-1) represents the peak area of the compound with Formula P-1.

Test Method 2: Calculation Method for Target Compound Yield

[Calculation Formula 2]

$$100 \times \{A(P\text{-}2) + A(P\text{-}1)\} / \text{Total peak area integrated including impurities}$$

The target compound yield is the percentage value of the actual target compound present in the reaction mixture, calculated according to [Calculation Formula 2] and predicted via HPLC. In the above formula, A represents the peak area of each compound in HPLC, determined using the method described below.

A(P-2) represents the peak area of the compound with Formula P-2;
A(P-1) represents the peak area of the compound with Formula P-1.

Test Method 3: Calculation Method for P-2:P-1 Ratio

[Calculation Formula 3]

$$100 * \frac{A(P-2)}{A(P-1)+A(P-2)} : 100 * \frac{A(P-1)}{A(P-1)+A(P-2)}$$

The P-2:P-1 ratio is the value calculated according to [Calculation Formula 3], representing the ratio of the compound with structure P-2 to the compound with structure P-1 among the target compounds (compounds with

structures P-2 and P-1) generated during the reaction, as predicted by HPLC.

In the above formula, A represents the peak area of each compound in HPLC, determined using the method described below.

A(P-2) represents the peak area of the compound with structure P-2;
A(P-1) represents the peak area of the compound with structure P-1.

**Comparative Example 1**

Comparative Example 1 shows a comparative oxidation reaction tested at various temperatures using the method described in Patent Document 4 (KR 10-2015-0120453 A), which was filed by German company PAION, however resulted in a rejection decision.

**Comparative Example 1-1: Same experiment as Example 3-1 of Patent Document 4**

The compound of Formula P-3 (100 mg, 0.22 mmol) was suspended in a mixed solvent of 0.77 mL methyl acetate and 1.3 mL toluene. To the reaction mixture, 14 wt% sodium hypochlorite aqueous solution (0.12 mL, 0.26 mmol), AZADO (0.112 mg, 0.00066 mmol), 0.41 mL of sodium hydrogen carbonate (7.7 wt%) aqueous solution, and potassium bromide (1.31 mg, 0.011 mmol) to the reaction mixture and stirred at 0°C for 1 hour. Subsequently, the oxidation conversion rate and the target compound were determined using HPLC according to Test Method 1 and Test Method 2 described above, respectively.

**Comparative Example 1-2: Experiment conducted at 15°C following Example 3-1 of Patent Document 4**

The compound of Formula P-3 (500 mg, 1.1 mmol) was suspended in a mixed solvent of 3.9 mL methyl acetate and 6.5 mL toluene. To the reaction mixture, 14 wt% sodium hypochlorite aqueous solution (0.6 mL, 1.3 mmol), AZADO (0.56 mg, 0.0033 mmol), 2.1 mL of sodium hydrogen carbonate (7.7 wt%) aqueous solution, and potassium bromide (6.6 mg, 0.055 mmol) to the reaction mixture and stirred at 15°C for 24 hours. The reaction mixture was analyzed by HPLC at 5 min, 30 min, 1 h, 2 h, 3 h, 5 h, 7 h, and 24 h after the start of the reaction. The oxidation conversion rate and the target compound were calculated according to Test Method 1 and Test Method 2 described above, respectively. Among the HPLC results for each time point, the result showing the highest target compound yield was plotted in Figure 1 below.

**Comparative Examples 1-3: Experiment conducted at Room Temperature following Example 3-1 of Patent Document 4**

The reaction was performed using the same method as Comparative Examples 1-2, except that the reaction temperature was changed to room temperature (25-30°C).

**Comparative Example 1-4: Experiment conducted at 40°C following Example 3-1 of Patent Document 4**

Except for changing the reaction temperature to 40°C, the method was the same as Comparative Example 1-2.

**Comparative Example 1-5: Experiment conducted at 50°C following Example 3-1 of Patent Document 4**

Except for changing the reaction temperature to 50°C, the method was the same as Comparative Example 1-2.

**Comparative Example 1-6: Experiment conducted at 60°C following Example 3-1 of Patent Document 4**

Except for changing the reaction temperature to 60°C, the method was the same as Comparative Example 1-2.

**Example 1: Comparison of reaction patterns upon replacing sodium hypochlorite aqueous solution with sodium hypochlorite pentahydrate**

Example 1 demonstrates that using sodium hypochlorite in its hydrated form is more effective for this oxidation reaction than the aqueous solution, by performing Comparative Example 1 using sodium hypochlorite pentahydrate.

**Experiment of Example 1-1:** Performed identically to Comparative Example 1-1, except substituting a 14 wt% sodium hypochlorite aqueous solution with an equivalent molar amount of sodium hypochlorite pentahydrate.

**Experiment of Example 1-2:** Performed in the same manner as Comparative Example 1-2, except that the 14

wt% sodium hypochlorite aqueous solution was replaced with an equivalent molar amount of sodium hypochlorite pentahydrate.

**Experiment of Example 1-3:** The experiment was performed in the same manner as Comparative Example 1-3, except that the 14 wt% sodium hypochlorite aqueous solution was replaced with an equivalent molar amount of sodium hypochlorite pentahydrate.

**Experiment of Example 1-4:** The experiment was performed in the same manner as Comparative Examples 1-4, except that the 14 wt% sodium hypochlorite aqueous solution was replaced with an equivalent molar amount of sodium hypochlorite pentahydrate.

**Experiment of Example 1-5:** Performed in the same manner as Comparative Examples 1-5, except that the 14 wt% sodium hypochlorite aqueous solution was replaced with an equivalent molar amount of sodium hypochlorite pentahydrate.

**Experiment for Example 1-6:** The experiment was performed using the same method as Comparative Examples 1-6, except that the 14 wt% sodium hypochlorite aqueous solution was replaced with an equivalent molar amount of sodium hypochlorite pentahydrate.

The results of Comparative Example 1 and Example 1 were compared by temperature and shown in Graph 1 of Figure 3.

Graph 1 shows that the results of Example 1 are superior across all temperature ranges from 0°C to 60°C. Example 1 exhibits optimal results with a target compound yield exceeding 90% in the 25°C-40°C range, corresponding to typical room temperature, indicating that roomtemperature reaction is possible without separate temperature control equipment. In contrast, the experimental results for Comparative Example 1 showed a low target compound yield of 30-40% in most temperature ranges. The optimum temperature among these was 15°C. Patent Document 4 (KR 10-2015 -0120453 A) also maintained a low temperature of 0°C to perform Comparative Example 1, indicating that a separate low-temperature maintenance device is required to use aqueous sodium hypochlorite in this reaction.

The target compound formation rates of Comparative Example 1 and Example 1, conducted at two temperatures (15°C and room temperature), are shown as a function of reaction time (Graph 2 in Figure 4 and Graph 3 in Figure 5). In Comparative Examples 1-3 using sodium hypochlorite aqueous solution, the target compound production rate sharply decreased within 2 hours at room temperature. In contrast, in Examples 1-3 using sodium hypochlorite hydrate, the product was maintained for over 24 hours. Comparative Examples 1-2 conducted at the lower temperature of 15°C showed a longer product retention time compared to Comparative Examples 1-3, but the product began to decrease after 7 hours, whereas the products in Examples 1-2 were maintained for over 24 hours. This demonstrates that while maintaining a low temperature is essential for product retention when using sodium hypochlorite as an aqueous solution in this oxidation reaction, the product remains stable even at room temperature when using the hydrate.

**Example 2:** Oxidation Reaction Using Calcium Peroxide (CaO2)/OXONE/TBAB Composite

Example 2 demonstrates that the calcium peroxide (CaO2)/OXONE/TBAB composite is effective as an auxiliary catalyst in the AZADO/hypochlorite oxidation reaction.

**Experiment of Example 2-1:** The experiment was conducted under conditions similar to Comparative Examples 1-3, except that the calcium peroxide (CaO2)/OXONE/TBAB composite was added.

After adding 14 wt% sodium hypochlorite aqueous solution (0.16 mL, 0.264 mmol) and AZADO (0.84 mg, 0.005 mmol) to 3 mL of acetonitrile, the mixture was stirred for 1 to 2 minutes at room temperature. P-3 (100 mg, 0.22 mmol), OXONE (298 mg, 0.484 mmol), TBAB (11.2 mg, 0.035 mmol), and calcium peroxide ($CaO_2$) (1.59 mg, 0.016 mmol) were added to the reaction mixture and stirred at room temperature for 30 minutes. Subsequently, the oxidation conversion rate and target compound yield were determined using HPLC according to Test Method 1 and Test Method 2 described above, respectively.

Oxidation conversion rate: 75.3% / Target compound yield: 55.9%

**Experiment for Example 2-2:** The experiment was performed similarly to Example 2-1, except that a 14 wt% sodium hypochlorite aqueous solution was replaced with an equivalent molar amount of sodium hypochlorite pentahydrate. The oxidation conversion rate and target compound yield were determined according to Test Method 1 and Test Method 2 described above, respectively.

Oxidation conversion rate: 98.2% / Target compound yield: 94.3%

[Table 1]

| | Comparative Example 1-3 | Example 1-3 | Example 2-1 | Example 2-2 |
|---|---|---|---|---|
| Sodium hypochlorite | Aqueous | 5 hydrate | Aqueous | 5 hydrate |
| Form | solution | | solution | |
| Use of combination (CaO2/OXONE/TBAB) | X | X | O | O |
| Result | % | | | |
| Oxidation conversion rate 30 minutes after reaction (Test Method 1) | 21.4 | 97.7 | 75.3 | 98.2 |
| Target compound yield after 30 minutes of reaction (Test Method 2) | 16.8 | 80.4 | 55.9 | 94.3 |

The above [Table 1] shows that the reaction is improved when using sodium hypochlorite pentahydrate and the composite agent ($CaO_2$/OXONE/TBAB).

The target compound yields of Comparative Example 1-3 and Examples 2-1, the target compound yield in Examples 1-3 and Example 2-2, where the sodium hypochlorite aqueous solution was replaced with the pentahydrate, increased by approximately 4.8-fold and 1.7-fold, respectively. This demonstrates that sodium hypochlorite pentahydrate is more effective than the aqueous solution in this oxidation reaction.

The results from Examples 2-1 and 2-2, where the complex agent was added to the respective conditions of Comparative Examples 1-3 and Examples 1-3, show an increase in the target compound yield by approximately 3-fold and 1.2-fold, respectively. This indicates that adding the complex agent improves this oxidation reaction.

**Example 3: Study on the Effect of Calcium Peroxide ($CaO_2$) Addition Amount**

**Experiment in Example 3-1:** No Calcium Peroxide ($CaO_2$) was added

Except for omitting calcium peroxide ($CaO_2$) addition, the same method as Example 2-2 was employed. The oxidation conversion rate and target compound yield were determined according to Test Methods 1 and 2 described above.

**Example 3-2 Experiment:** Using 3.7 mol% calcium peroxide ($CaO_2$)

The method was performed identically to Example 2-2, except that the amount of calcium peroxide ($CaO_2$) used was changed to (3.7 mol%, 0.0082 mmol, 0.79 mg). The oxidation conversion rate and the yield of the target compound were determined according to Test Method 1 and Test Method 2 described above, respectively.

**Experiment of Example 3-3:** Using 10 mol% calcium peroxide ($CaO_2$)

The method was performed identically to Example 2-2, except that the amount of calcium peroxide ($CaO_2$) used was changed to 10 mol% (0.022 mmol, 2.11 mg). The oxidation conversion rate and target compound yield were determined according to Test Method 1 and Test Method 2 described above, respectively. An aqueous solution of sodium thiosulfate was then added to the reaction mixture, causing it to separate into layers. The resulting organic layer was washed with an aqueous solution of ammonium chloride. The obtained organic layer was dried over anhydrous sodium sulfate, filtered, and the filtrate was concentrated under reduced pressure.

**Experiment for Examples 3-4:** Using 15 mol% calcium peroxide ($CaO_2$)

The method was performed identically to Example 2-2, except that the calcium peroxide ($CaO_2$) amount was changed to (15 mol%, 0.033 mmol, 3.17 mg). The oxidation conversion rate and target compound yield were determined according to Test Method 1 and Test Method 2 described above, respectively.

The results of Examples 3-1 to 3-4 were compared with those of Example 2-2 and are shown in Table 2 below.

[Table 2]

| | Example 3-1 | Example 3-2 | Example 2-2 | Example 3-3 | Example 3-4 |
|---|---|---|---|---|---|
| Calcium peroxide ($CaO_2$) Used equivalent amount | - | 3.7 mol% | 7.5 mol% | 10 mol% | 15 mol% |
| Oxidation conversion rate (Test method 1) | 95.3 | 96.6 | 98.2 | 98.9 | 92.2 |
| Target compound yield (Test method 2) | 82.2 | 88.4 | 94.3 | 91.5 | 86.7 |
| P-2:P-1 ratio (Test method 3) | 56:44 | 49:51 | 60:40 | 41:59 | 59:41 |

From the results in Table 2, it was confirmed that when calcium peroxide ($CaO_2$) was excluded (Example 3-1), both the oxidation conversion rate and the target compound yield were the lowest. This confirmed that calcium peroxide ($CaO_2$) not only induces a stable oxidation reaction in this oxidation reaction but also reduces impurity formation.

However, this oxidation efficiency did not increase proportionally with the equivalent amount of calcium peroxide ($CaO_2$). Instead, maximum efficiency was observed at an appropriate amount. Furthermore, the ratio of P-1 generated during the reaction also varied with the equivalent amount of calcium peroxide ($CaO_2$). When the equivalent of calcium peroxide ($CaO_2$) was approximately 10 mol% (Example 3-3), the oxidation reaction showed maximum conversion, and the ratio of P-1 was also the highest.

**Example 4: Synthesis of remimazolam besylate from the Product of Example 3-3**

Benzene sulfonic acid (38.3 mg, 0.24 mmol) was added to the concentrated residue from Example 3-3, along with a mixed solvent of methanol and ethyl acetate (mixing ratio 1:30), and stirred overnight at room temperature. The reaction mixture was filtered with 100% ethyl acetate, and the solid obtained was dissolved in methanol. Activated carbon (30 mg) was added, stirred at room temperature for 2 hours, and then filtered. The filtrate was redissolved in a mixed solvent of MeOH and EA (mixing ratio: 1:10) and stirred at room temperature overnight. The precipitated crystals were filtered and washed to yield 124.3 mg of remimazolam besylate (P). (Yield: 95%, Purity: 98.49%) The obtained remimazolam besylate was analyzed by HPLC, as shown in Figure 2 below.

**Example 5: Oxidation Reaction using ABNO**

Example 5 demonstrates that various N-oxyl oxidation catalysts can be used in this oxidation reaction by employing ABNO, which functions similarly to AZADO, instead of AZADO.

**Experiment of Example 5-1:** Sodium hypochlorite pentahydrate (2.7 g, 16.3 mmol) was suspended in 90 mL of tetrahydrofuran. ABNO (96.4 mg, 0.65 mmol) and the compound of Formula P-3 (3.0 g, 6.5 mmol) were added to the reaction mixture, stirred at room temperature for 1 hour, and then the oxidation conversion rate and target compound yield were determined using HPLC according to Test Method 1 and Test Method 2 described above, respectively. Subsequently, 90 mL of 20% sodium thiosulfate aqueous solution was added, stirred for 30 minutes, and then 45 mL of ethyl acetate was further added and stirred for 30 minutes. The separated organic layer was extracted using a separating funnel, dried over sodium sulfate, and concentrated using a rotary evaporator.
Oxidation conversion rate: 99.1% / Target compound yield: 98.0%

**Experiment for Example 5-2: Synthesis of Remimazolam Besylate from the Product of Example 5-1**

516 mg (3.3 mmol) of benzenesulfonic acid, 18 mL of a mixed solvent of ethanol and ethyl acetate (mixing ratio 1:5) were added to the concentrated residue from Example 5-1, and the mixture was stirred overnight at room temperature. The precipitated solid was filtered and washed with the same solvent as the reaction solvent, yielding 1.57 g of remimazolam besylate (P). (yield: 81%, purity: 98%)

[Table 3]

| | Comparative Example 1-1 | Example 1-3 | Example 3-3 | Example 5-1 |
|---|---|---|---|---|
| Components | Equivalent (eq)/mmol/· amount used | | | |
| Formula P-3 | 1.0 eq, 0.22 mmol, 100 mg | 1.0 eq, 1.1 mmol, 500 mg | 1.0 eq, 0.22 mmol, 100 mg | 1.0 eq, 6.53 mmol, 3.0 g |
| N-oxyl oxidation catalyst | (AZADO) 0.3 mol%, 0.00066 mmol, 0.112 mg | (AZADO) 0.3 mol%, 0.0033 mmol, 0.56 mg | (AZADO) 2.3 mol%, 0.005 mmol, 0.84 mg | (ABNO) 10 mol%, 0.65 mmol, 96.4 mg |
| $NaOCl\cdot 5H_2O$ | | 1.2eq, 1.3mmol, 215mg | 1.2 eq, 0.264 mmol, 43 mg | 2.5 eq, 16.3 mmol, 2.7 g |
| 14% NaOCl | 1.2 eq, 0.26 mmol, 0.12 mL | | - | - |
| Sodium bicarbonate ($NaHCO_3$) | (7.7 wt% Aq. solution 0.41 mL | (7.7 wt% Aq. solution 2.1 mL | - | - |
| NaBr/KBr | (KBr) 5 mol%, 0.011 mmol, 1.31 mg | (KBr) 5 mol%, 0.055 mmol, 6.6 mg | - | - |
| Calcium peroixde ($CaO_2$) | | | 10 mol%, 0.022 mmol, 2.11 mg | - |
| OXONE | | | 2.2 eq, 0.484 mmol, 298 mg | - |
| TBAB | | | 16 mol%, 0.0352 mmol, 11.2 mg | - |
| Solvent | MeOAc 0.77 mL, Toluene 1.3 mL | MeOAc 0.77 mL, Toluene 1.3 mL | Acetonitrile 3 mL | Tetahydrofuran |
| Temp./time | 0°C, 1 hr | Room temp., 30 min | Room temp., 30 min | Room temp., 1 hr |
| Result | % | | | |
| Oxidation conversion rate (Test method 1) | 32.8% | 97.7% | 98.9 % | 99.1 % |
| Target Compound yield (Test method 2) | 30.5% | 80.4% | 91.5 % | 98.0 % |
| P-2:P-1 ratio (Test method 3) | 65.2:34.7 | 2.3:97.7 | 41:59 | 63:36 |

Table 3, comparing experiments on prior art comparable to the present invention (Comparative Example 1-1) with Examples 1-3, 3-3, and 5-1, demonstrates that the use of sodium hypochlorite pentahydrate significantly improves the oxidation efficiency and reaction conditions of the present invention. The effectiveness of the compound can be explained by the fact that it eliminates the need for low-temperature maintenance and pH adjustment, and increases the yield of the target compound by more than threefold within a reaction time of 30 minutes.

Furthermore, the ratio of compound P-1, calculated according to Test Method 3, is more favorable than that of Comparative Example 1-1, making it more advantageous for the subsequent cyclization reaction and the synthesis of remimazolam besylate. The P-2 : P-1 ratio and the degree of impurity generation in the reaction solution of Example 3-3, analyzed by HPLC according to Test Method 3, are shown in Figure 1 below.

[Table 4]

| | Patent Document 1 | Patent Document 3 | Example 4 | Example 5-2 |
|---|---|---|---|---|
| Yield | 50% | 58.43% | 95% | 81% |

Examples 4 and Example 5-2 demonstrate a significant increase in overall yield compared to prior art when remimazolam besylate is synthesized from the products obtained under the optimal conditions of the present invention, as shown in Example 3-3 and Example 5-1. The results in Table 4 represent the purification yields for the two steps of manufacturing remimazolam besylate from the compound of Formula P-3. The results for Patent Document 1 and Patent Document 3 were referenced from the yields reported in the literature. The results from Patent Document 4 were unavailable, so we conducted our own experiments. However, the yield was low during the oxidation step (oxidation conversion rate: 32.8%), preventing us from proceeding to the final reaction.

## Claims

**1.** A method for preparing remimazolam besylate of 3-[(4S)-8-Bromo-1-methyl-6-(2-pyridinyl)-4H-imidazo[1,2-a][1,4] benzodiazepin-4-yl]propionic acid methyl ester benzenesulfonate having the following Formula P, the method comprising steps of

(a) oxidating 3-((3S)-7-bromo-2-((2-hydroxypropyl)amino)-5-(pyridin-2-yl)-3H-benzo[e][1,4]diazepin-3-yl)propionic acid methyl ester of Formula P-3 with a hydrated hypochlorite salt and N-oxyl oxidizing catalyst in the presence of an organic solvent; and
(b) treating the reaction product obtained in step (a) with benzenesulfonic acid in an organic solvent;

[P-3]

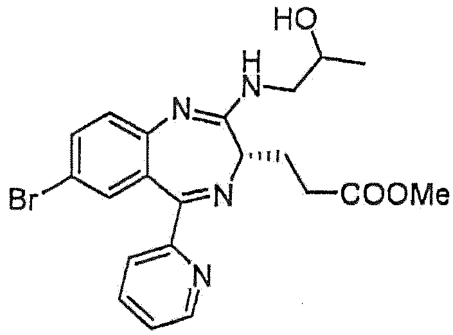

[P]

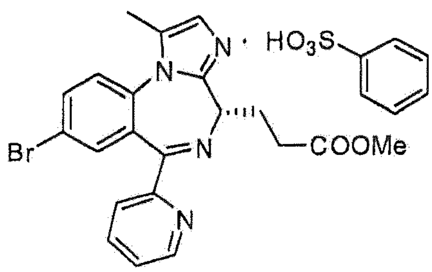

**2.** The method according to Claim 1, wherein the hydrate of the hypochlorite salt is at least one selected from the group consisting of the hydrate of sodium hypochlorite (NaOCl), the hydrate of potassium hypochlorite (KOCl), and the hydrate of calcium hypochlorite ($Ca(OCl)_2$).

**3.** The method according to Claim 1, wherein the hydrate of the hypochlorite salt is sodium hypochlorite pentahydrate ($NaOCl \cdot 5H_2O$).

**4.** The method according to Claim 1, wherein the hypochlorite salt hydrate is used in an equivalent ratio of 0.1 to 5 relative to the compound represented by Formula P-3.

**5.** The method according to Claim 1, wherein the N-oxyl oxidation catalyst is at least one selected from the group consisting of

2-azaadamantane-N-oxyl (AZADO) represented Formula 1,
1-methyl-2-azaadamantane-N-oxyl (Me-AZADO) represented by Formula 2,
2-hydroxy-2-azaadamantane (AZADOH) represented by Formula 3,
2,2,6,6, tetramethylpiperidine N-oxyl (TEMPO) represented by Formula 4,
And 9-azabicyclo[3.3.1]nonan-N-oxyl (ABNO) represented by Formula 5, their salts and their solvates:

[Formula 1]

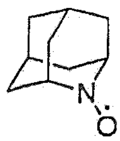

[Formula 2]

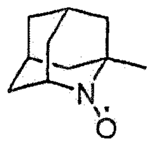

[Formula 3]

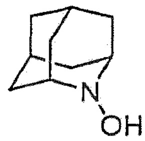

[Formula 4]

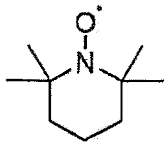

[Formula 5]

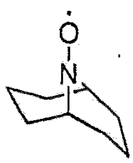

**6.** The method according to Claim 1, wherein N-oxyl oxidation catalyst is used in equivalent ratio of 0.01 to 0.2 relative to the compound of Formula P-3.

**7.** The method according to Claim 1, wherein an ammonium salt is additionally used in step (a).

**8.** The method according to Claim 7, wherein the ammonium salt is at least one selected from the group consisting of tetrabutylammonium bromide (TBAB), tetrabutylammonium fluoride (TBAF), tetrabutylammonium chloride (TBAC), tetrabutylammonium iodide (TBAI), their salts, and their solvates.

**9.** The method according to Claim 7, wherein the ammonium salt is used in an amount of 0.01 to 0.5 equivalent ratio relative to the compound of Formula P-3.

**10.** The method according to Claim 1, wherein an inorganic oxidizing agent is additionally used in step (a).

**11.** The method according to Claim 10, wherein the inorganic oxidizing agent is at least one selected from the group consisting of oxone ($2KHSO_5.KHSO_4.K_2SO_4$), its salts, and its solvates.

**12.** The method according to Claim 10, wherein the inorganic oxidizing agent is not used for the compound of Formula P-3, or is used in an equimolar ratio of 0.01 to 3.

**13.** The method according to Claim 1, wherein an alkaline earth metal peroxide is additionally used in step (a).

**14.** The method according to Claim 13, wherein the peroxide of the alkaline earth metal is at least one selected from the group consisting of calcium peroxide ($CaO_2$), magnesium peroxide ($MgO_2$), salts thereof, and solvates thereof.

**15.** The method according to Claim 13, wherein the peroxide of an alkaline earth metal is not used for the compound of Formula P-3, or is used in an equimolar ratio of 0.01 to 0.5.

**16.** The method according to any one of Claims 1-15, wherein the organic solvent used in step (a) is selected from ethyl acetate, methyl acetate, methylene chloride, dichloroethane, toluene, acetone, diethyl ether, methyl tert-butyl ether, pentane, hexane, acetonitrile, tetrahydrofuran, and mixtures thereof.

**17.** The method according to any one of Claims 1-15, wherein after the oxidation reaction in step (a) proceeds with a conversion rate of 98% or more, step (b) is performed using the product obtained by working up the reaction mixture.

**18.** The method according to Claim 17, wherein the oxidation reaction in step (a) is carried out within 15 to 180 minutes.

**19.** The method according to any one of Claims 1-15, wherein the oxidation reaction in step (a) is carried out within the range of 0 to 30°C.

**20.** The method according to any one of Claims 1-15, wherein in step (b), the organic solvent is selected from the group consisting of ethyl acetate, methyl acetate, methanol, ethanol, methyl ethyl ketone, and mixtures thereof.

**21.** The method according to any one of Claims 1-15, wherein in step (b), benzene sulfonic acid is used in an amount of 0.9 to 1.1 equivalents relative to the compound of Formula P-3.

**FIGURE 1**

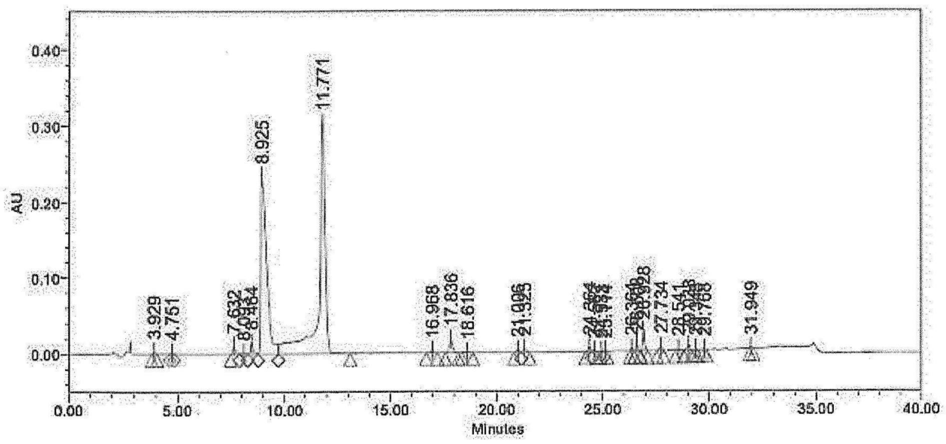


**Peak Results**

| | Name | RT | Area | % Area | Height | Amount | Units |
|---|---|---|---|---|---|---|---|
| 1 | | 3.929 | 14183 | 0.12 | 2924 | | |
| 2 | | 4.751 | 9851 | 0.08 | 1827 | | |
| 3 | | 7.632 | 78110 | 0.66 | 10750 | | |
| 4 | | 8.093 | 12212 | 0.10 | 1028 | | |
| 5 | | 8.464 | 125238 | 1.06 | 14941 | | |
| 6 | | 8.925 | 4497761 | 37.98 | 233802 | | |
| 7 | | 11.771 | 6340338 | 53.54 | 303333 | | |
| 8 | | 16.968 | 26954 | 0.23 | 2482 | | |
| 9 | | 17.836 | 183547 | 1.55 | 17596 | | |
| 10 | | 18.616 | 6231 | 0.05 | 433 | | |

| | Name | RT | Area | % Area | Height | Amount | Units |
|---|---|---|---|---|---|---|---|
| 11 | | 21.006 | 38555 | 0.33 | 3588 | | |
| 12 | | 21.325 | 57970 | 0.49 | 5096 | | |
| 13 | | 24.364 | 46013 | 0.39 | 5741 | | |
| 14 | | 24.582 | 14730 | 0.12 | 2011 | | |
| 15 | | 24.933 | 6610 | 0.06 | 1014 | | |
| 16 | | 25.114 | 7821 | 0.07 | 1188 | | |
| 17 | | 26.364 | 19767 | 0.17 | 3535 | | |
| 18 | | 26.608 | 64913 | 0.55 | 10444 | | |
| 19 | | 26.928 | 153806 | 1.30 | 24604 | | |
| 20 | | 27.734 | 46377 | 0.39 | 5625 | | |

| | Name | RT | Area | % Area | Height | Amount | Units |
|---|---|---|---|---|---|---|---|
| 21 | | 28.541 | 16788 | 0.14 | 1735 | | |
| 22 | | 29.035 | 48194 | 0.41 | 6497 | | |
| 23 | | 29.348 | 8418 | 0.07 | 1695 | | |
| 24 | | 29.768 | 8979 | 0.07 | 1458 | | |
| 25 | | 31.949 | 7810 | 0.07 | 1597 | | |

**FIGURE 2**

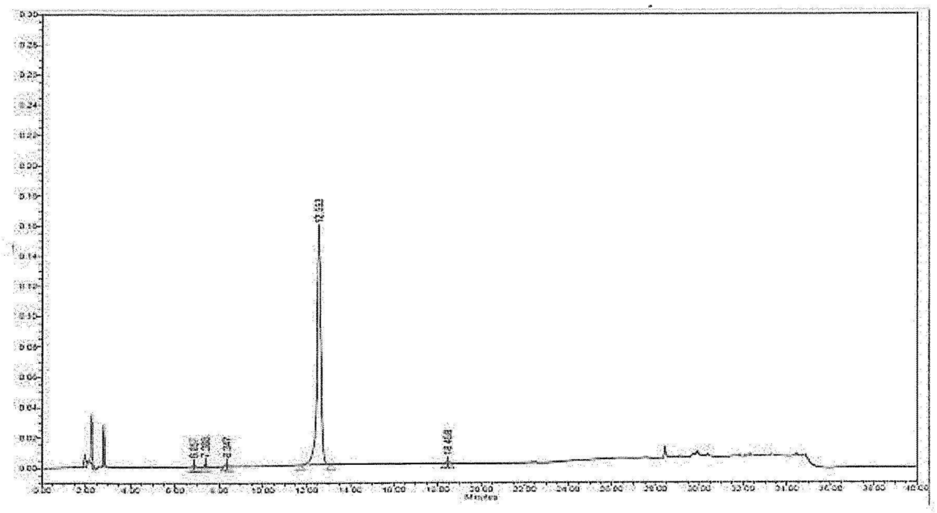

**Peak Results**

| | Name | RT | Area | % Area | Height | Amount | Units |
|---|---|---|---|---|---|---|---|
| 1 | | 6.857 | 6583 | 0.33 | 1353 | | |
| 2 | | 7.388 | 7435 | 0.38 | 1628 | | |
| 3 | | 8.347 | 8293 | 0.42 | 1453 | | |
| 4 | | 12.553 | 1939997 | 98.49 | 155507 | | |
| 5 | | 18.458 | 7347 | 0.37 | 964 | | |

## FIGURE 3

[ Graph 1]

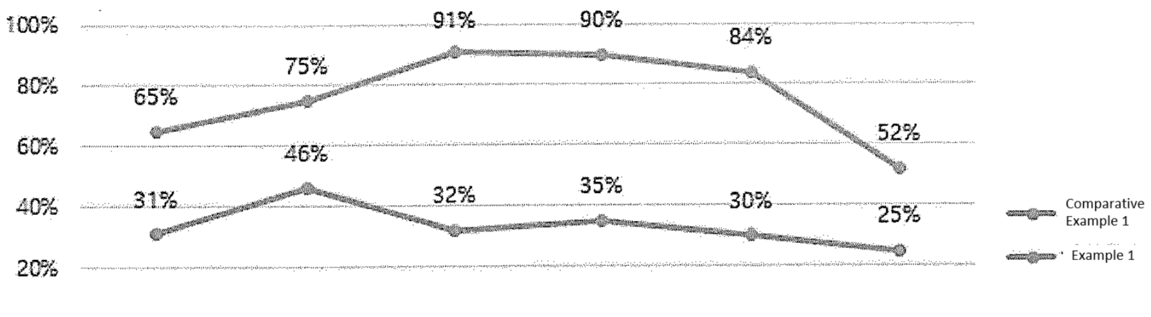


| | 0℃ | 15℃ | 실온(25-30℃) | 40℃ | 50℃ | 60℃ |
|---|---|---|---|---|---|---|
| Comparative Example 1 | 31% | 46% | 32% | 35% | 30% | 25% |
| Comparative Example 1 | 65% | 75% | 91% | 90% | 84% | 52% |

## FIGURE 4

[Graph 2] Reaction temperature : 15℃

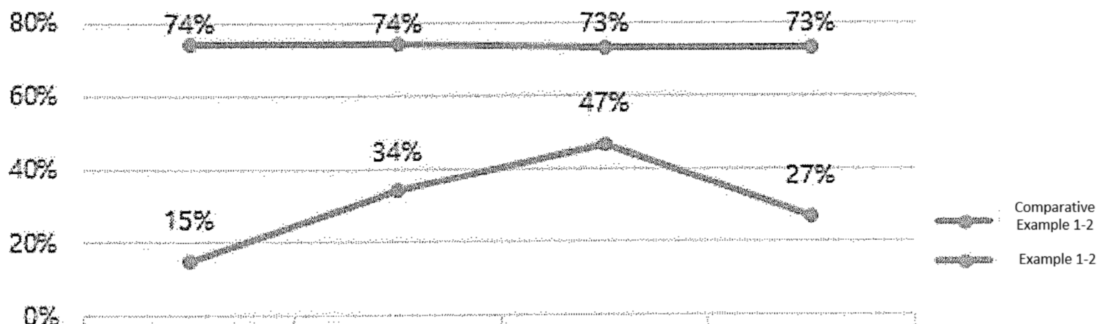


| | 30 min | 2 hr | 7 hr | 24 hr |
|---|---|---|---|---|
| Comparative Example 1-2 | 15% | 34% | 47% | 27% |
| Example 1-2 | 74% | 74% | 73% | 73% |

## FIGURE 5

[Graph 3] Reaction temperature: Room temperature

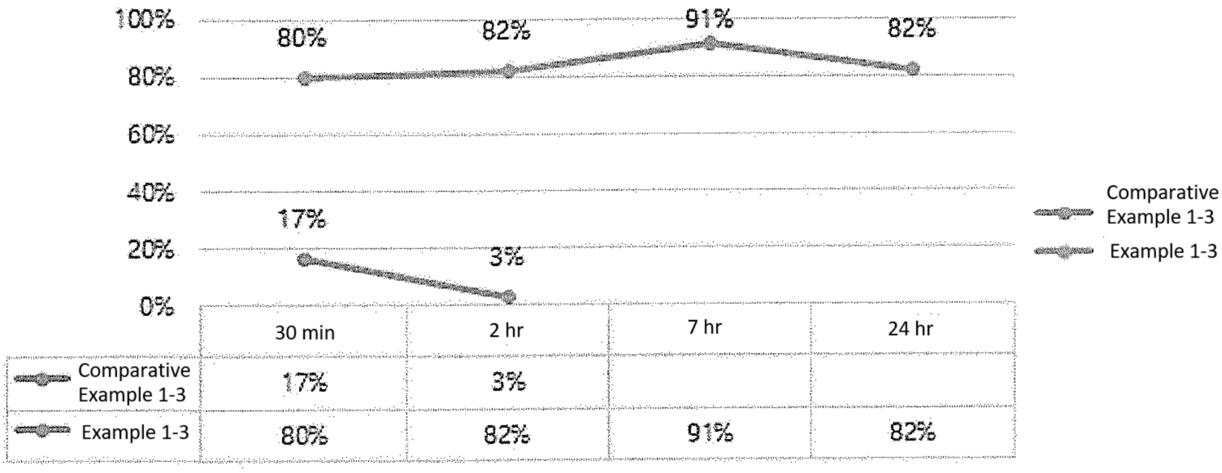


| | 30 min | 2 hr | 7 hr | 24 hr |
|---|---|---|---|---|
| Comparative Example 1-3 | 17% | 3% | | |
| Example 1-3 | 80% | 82% | 91% | 82% |

## INTERNATIONAL SEARCH REPORT

International application No.
**PCT/KR2024/006939**

**A. CLASSIFICATION OF SUBJECT MATTER**

**C07D 487/04**(2006.01)i; **A61K 31/5517**(2006.01)i; **A61P 25/20**(2006.01)i

According to International Patent Classification (IPC) or to both national classification and IPC

**B. FIELDS SEARCHED**

Minimum documentation searched (classification system followed by classification symbols)

C07D 487/04(2006.01); A61K 31/5517(2006.01); C07C 45/50(2006.01); C07C 49/04(2006.01); C07D 213/40(2006.01); C07D 401/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 3-[(S)-7-브로모-2-(2-하이드록시-프로필아미노)-5-피리딘-2-일-3H-벤조[e][1,4]디아제핀-3-일]프로피온산 메틸 에스테르 (3-[(S)-7-bromo-2-(2-hydroxy-propylamino)-5-pyridine-2-yl-3H-benzo[e][1,4]diazepine-3-yl]propionic acid methyl ester), 차아염소산 수화물 (hypochlorous acid hydrate), 레미마졸람베실산염 (remimazolam besylate)

**C. DOCUMENTS CONSIDERED TO BE RELEVANT**

| Category* | Citation of document, with indication, where appropriate, of the relevant passages | Relevant to claim No. |
|---|---|---|
| DX | KR 10-2015-0120453 A (PAION UK LIMITED) 27 October 2015 (2015-10-27)<br>See claims 9, 13 and 14; paragraphs [0019], [0161]-[0163], [0176], [0214], [0215], [0274] and [0326]; and examples 2, 3 and 6. | 1,2,4-12,16-21 |
| DA | | 3,13-15 |
| DA | KR 10-2021-0157146 A (HANA PHARM. CO., LTD.) 28 December 2021 (2021-12-28)<br>See entire document. | 1-21 |
| DA | CN 112321594 A (YANGTZE RIVER PHARMACEUTICAL GROUP CO., LTD.) 05 February 2021 (2021-02-05)<br>See entire document. | 1-21 |
| DA | KR 10-2012-0102603 A (PAION UK LIMITED) 18 September 2012 (2012-09-18)<br>See entire document. | 1-21 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 September 2024** | **04 September 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

INTERNATIONAL SEARCH REPORT

International application No.
PCT/KR2024/006939

C. DOCUMENTS CONSIDERED TO BE RELEVANT

| Category* | Citation of document, with indication, where appropriate, of the relevant passages | Relevant to claim No. |
|---|---|---|
| A | KR 10-2015-0026729 A (NIPPON LIGHT METAL COMPANY, LIMITED) 11 March 2015 (2015-03-11)<br>See entire document. | 1-21 |

Form PCT/ISA/210 (second sheet) (July 2022)

**INTERNATIONAL SEARCH REPORT**
**Information on patent family members**

International application No.
**PCT/KR2024/006939**

| Patent document cited in search report | Publication date (day/month/year) | Patent family member(s) | Publication date (day/month/year) |
|---|---|---|---|
| KR 10-2015-0120453 A | 27 October 2015 | AR 094963 A1 | 09 September 2015 |
| | | AU 2014-227095 A1 | 08 October 2015 |
| | | BR 112015021007 A2 | 18 July 2017 |
| | | CA 2902631 A1 | 12 September 2014 |
| | | CN 105246881 A | 13 January 2016 |
| | | EA 032065 B1 | 30 April 2019 |
| | | EA 201591542 A1 | 30 December 2015 |
| | | EP 2966069 A1 | 13 January 2016 |
| | | EP 2966069 A4 | 30 November 2016 |
| | | EP 2966069 B1 | 08 January 2020 |
| | | GE P20186886 B | 10 August 2018 |
| | | HK 1212991 A1 | 24 June 2016 |
| | | IL 240890 B | 30 April 2018 |
| | | JP 2017-122116 A | 13 July 2017 |
| | | JP 2017-136730 A1 | 09 February 2017 |
| | | MX 2015011431 A | 03 February 2016 |
| | | TW 201443035 A | 16 November 2014 |
| | | UA 119138 C2 | 10 May 2019 |
| | | US 10414749 B2 | 17 September 2019 |
| | | US 2016-0009680 A1 | 14 January 2016 |
| | | US 2017-0217925 A1 | 03 August 2017 |
| | | US 2018-0141928 A1 | 24 May 2018 |
| | | US 9656987 B2 | 23 May 2017 |
| | | US 9981941 B2 | 29 May 2018 |
| | | WO 2014-136730 A1 | 12 September 2014 |
| KR 10-2021-0157146 A | 28 December 2021 | KR 10-2526590 B1 | 28 April 2023 |
| | | WO 2021-256679 A1 | 23 December 2021 |
| CN 112321594 A | 05 February 2021 | CN 112321594 B | 20 May 2022 |
| KR 10-2012-0102603 A | 18 September 2012 | AU 2010-294873 A1 | 12 April 2012 |
| | | AU 2010-294873 B2 | 13 November 2014 |
| | | BR 112012006053 A2 | 03 November 2020 |
| | | BR 112012006053 B1 | 16 November 2021 |
| | | CA 2774018 A1 | 24 March 2011 |
| | | CA 2774018 C | 30 January 2018 |
| | | CN 102753525 A | 24 October 2012 |
| | | CN 102753525 B | 02 September 2015 |
| | | DK 2477967 T3 | 21 December 2015 |
| | | EP 2305647 A1 | 06 April 2011 |
| | | EP 2477967 A1 | 25 July 2012 |
| | | EP 2477967 B1 | 11 November 2015 |
| | | ES 2555233 T3 | 29 December 2015 |
| | | HK 1173437 A1 | 16 May 2013 |
| | | HR P20151316 T1 | 15 January 2016 |
| | | HU E028298 T2 | 28 December 2016 |
| | | JP 2013-505207 A | 14 February 2013 |
| | | JP 5801309 B2 | 28 October 2015 |
| | | KR 10-1801735 B1 | 27 November 2017 |
| | | MX 2012003183 A | 08 May 2012 |
| | | MX 337683 B | 15 March 2016 |
| | | MX 341093 B | 08 August 2016 |

Form PCT/ISA/210 (patent family annex) (July 2022)

**INTERNATIONAL SEARCH REPORT**
**Information on patent family members**

International application No.
**PCT/KR2024/006939**

| Patent document cited in search report | Publication date (day/month/year) | Patent family member(s) | Publication date (day/month/year) |
|---|---|---|---|
| | | PL 2477967 T3 | 29 April 2016 |
| | | PT 2477967 E | 07 January 2016 |
| | | RU 2012115456 A | 10 November 2013 |
| | | RU 2551848 C2 | 27 May 2015 |
| | | US 10000464 B2 | 19 June 2018 |
| | | US 2012-0330007 A1 | 27 December 2012 |
| | | US 2015-0368199 A1 | 24 December 2015 |
| | | US 2017-0044135 A1 | 16 February 2017 |
| | | US 9156842 B2 | 13 October 2015 |
| | | US 9512078 B2 | 06 December 2016 |
| | | WO 2011-032692 A1 | 24 March 2011 |
| KR 10-2015-0026729 A | 11 March 2015 | JP 2015-063504 A | 09 April 2015 |
| | | JP 6176177 B2 | 09 August 2017 |
| | | TW 201509894 A | 16 March 2015 |
| | | TW I596086 B | 21 August 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 101801735 B
- KR 1020210157146 A
- CN 112321594 B
- KR 1020150120453 A
- KR 101162648 B


### Non-patent literature cited in the description


- *Organic Process Research & Development*, 2017, vol. 21 (12), 1925-1937
- *Molecules*, 2020, vol. 25, 5918
- *CHEMICAL ABSTRACTS*, 71878-19-8